# EUROPEAN PATENT SPECIFICATION

(11) **EP 2 509 680 B1**
(45) Date of publication and mention of the grant of the patent: **16.09.2015**
(21) Application number: 10809051.5
(22) Date of filing: 07.12.2010
(51) Int. Cl.: A61N 1/30, A61N 1/44, H01T 23/00

(54) **THERAPEUTIC DEVICE**
THERAPEUTISCHE VORRICHTUNG
DISPOSITIF THÉRAPEUTIQUE

(30) Priority: 09.12.2009 IT MO20090287
(43) Date of publication of application: 17.10.2012
(73) Proprietor: BIOS OMNIA S.R.L, 20134 Milano (IT)
(72) Inventor: MAURO, Giuseppe Mario, 20134 Milano (IT)
(74) Representative: Bergamini, Silvio
(86) International application number: PCT/IB2010/055613
(87) International publication number: WO 2011/070500

(56) References cited:
- EP-A1- 1 016 433
- FR-A1- 2 287 791
- GB-A- 1 167 053
- US-A- 2 132 539
- US-A- 3 417 302
- US-A- 3 624 448
- US-A- 4 435 195
- US-A- 5 628 729
- US-A1- 2003 108 460

## Description

### Field of the invention

The present invention relates to a therapeutic device, namely a portable device for treating diseases and disorders by iontophoresis.

### Background art

Iontophoresis (or galvano therapy) has been long known to be used for treating physical discomfort, such as diseases and disorders and providing a sense of well-being and quietness to human beings.

Iontophoresis basically consists in electric generation of ions, generally negative ions, in the form of a flow designed to impinge upon regions of the body of a patient undergoing treatment.

It is particularly suitable for attenuating pain of various types, such as those caused by trauma or inflammations of joints or arthritis, arthrosis, sciatica, backache, torn muscles, and the like. Furthermore, iontophoresis is also used to induce relaxation in muscle masses and hence also psychological relaxation, to improve the effectiveness of medicaments when administered in combination with ionic therapy, to improve breathing and hence oxygenation of the entire body, particularly the brain, which is thus kept particularly active and alert.

In practice, ions move between one positive pole and one negative pole of a special device, thereby creating a flow of migrating ions through the body, or a part thereof, for transcutaneous administration of medicaments and active ingredients thereof in considerably smaller amounts as compared with conventional, e.g. oral administration, no overdosage being required to account for the dispersion of part of the drugs occurring through the body before reaching the target area or point.

A number of professional devices have been known for carrying out this therapy, which can be used both in hospitalized patients and in domestic settings.

The latter are particularly appreciated for their simple construction and use, their portability and their relatively low cost, as compared with professional devices.

These ion emitter devices basically consist of a ion generator, which is connected, by means of respective power-carrying wires, with a pair of terminal electrodes designed to be placed on the part of the patient body requiring treatment to reduce or eliminate a painful condition.

The generator is typically equipped with a controller, which is adapted to control the intensity of the emitted electric current and the duration of teratment cycles.

One of the electrodes forms an anode, and the other electrode forms a cathode, and the medicaments to be administered by iontophoresis are previously applied to the anode or the cathode, depending on the polarity of the active ingredient of the drug.

A further version of a device for iontophoretic treatments comprises a mat, whose size changes as a function of the size of the area to be treated, in which a flat and flexible electrode, i.e. either the cathode or the anode, can be placed, whereas the electrode of opposing polarity consists of the element for connection to the patient's body.

Here, for treatment to be performed, the patient shall lie on the mat and actuate the ion generator, to be impinged upon by a flow generated thereby between the cathode and the anode.

This prior art has provided and still provides satisfactory results in terms of ionic treatment effectiveness, nevertheless the apparatus that are used for treatment are substantially inconvenient to use as they either involve local and substantially accurate application of electrodes, or require the patient to lie on a radiating mat.

A further device providing iontophoresis is disclosed in US 2003/0108460 A1.

Therefore, in either case, the patient must to stop its physical and work activities for the time required to complete the therapeutic cycle.

### Disclosure of the invention

It is an object of the present invention to improve the prior art.

Another object is to provide a therapeutic device that allows iontophoretic treatment of patients even without requiring them to stop their physical or work activities.

Yet another object of the invention is to provide a therapeutic device that has a very simple and inexpensive construction.

A further object of the invention is to provide a therapeutic device that is portable and allows any positioning option, the term positioning involving that it can either lie on a surface or hang from a support or a wall or the like.

The invention is defined in the appended set of claims.

In one aspect, the invention provides a portable therapeutic device, wherein the device comprises: a ion generator arrangement; an emitter apparatus for emitting negative ions (anions) toward the body of a patient, which is connected to said generator arrangement by a connecting wire and defines a first pole having a negative polarity; an electrode having a positive polarity , which defines a second pole connected to said body of a patient, wherein said emitter apparatus comprises a net element which defines net meshes and nodes for connection of said net meshes, said nodes being emitting points for ion flows toward said second pole through said body of a patient.

Therefore, the invention provides the following advantages:
convenient application of iontophoretic treatments, without requiring any interruption of physical and/or work activities;
provision of a simple and inexpensive therapeutic device, as compared with those that are currently available;
easy transportation of the therapeutic device wherever it is required to be used;
self-standing orientation of the therapeutic device in a predetermined radiating direction, without requiring it to be hand-held or applied to a part of the body.

### Brief description of the drawings

Further features and advantages of the invention will be more readily apparent upon reading of the detailed description of a preferred non exclusive embodiment of a therapeutic device, which is shown as a non limiting example by the annexed drawings, in which:
FIG. 1 is a front schematic view of an emitter apparatus of the therapeutic device of the invention;
FIG. 2 is a cross-sectional view of the emitter apparatus of Fig. 1, as taken along a plane *II* - *II;*
FIG. 3 is a schematic view of a step of use of the therapeutic device of the invention by a patient.

### Detailed description of one preferred embodiment

Referring to the above mentioned figures, a therapeutic device is generally designated by numeral 1 and comprises a ion generator arrangement 2.

The generator arrangement 2 is connected by means of a connecting wire 3 to an emitter apparatus for emitting flows of negative ions, or anions, from the generator arrangement 2, which is generally designated by numeral 4 and, as shown in a possible and preferred embodiment, is provided in the form of a flat screen 5.

This flat screen 5 comprises a net element 6 made of a conductive material which forms a multiplicity of meshes to define node points 7 at which the wires that form the meshes of the net element 6 intersect, which points are emitting points for the ions generated by the generator arrangement 2, and create a ionic flow designated by the arrows "F".

The generator arrangement 2 is also connected with a patient "P" that undergoes the iontophoretic treatment, by means of a second connecting wire 8 associated with the patient "P".

In the embodiment of the therapeutic device 1, the screen 5 is connected with the cathode "C" of the generator arrangement 2, whereas the patient "P" is connected with the anode "A".

As shown in the figures, the screen 5 has lower feet 10 for lying on a surface.

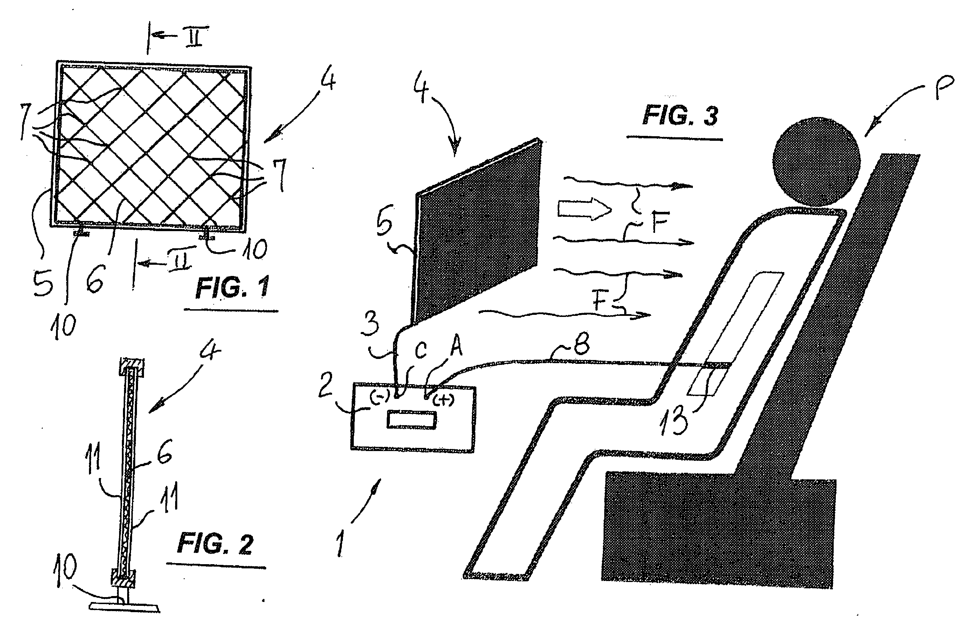

Furthermore, referring to Fig. 2, the net element 6 is shown to be interposed between two plastic containing sheets 11, which may be, for instance, two ABS (ACRYLONITRILE - BUTADIENE - STYRENE) or clear, matt or colored polystyrene sheets.

The operation of the therapeutic device 1 is as follows: when a patient "P" is to undergo a ion radiation treatment, the screen 5 is placed in the predetermined position, depending on the direction that the ion flows "F" have to follow toward the patient "P", by placing it with the feet 10 on a load-bearing surface.

Then, the free end of the cable 8, possibly equipped with a bracelet 13, is connected with the patient "P", and the generator arrangement 2 is actuated, with the desired emission time and intensity settings, whereupon the arrangement transmits the generated negative ions (anions) to the net element 6, the ions being projected toward the patient "P" by being attracted toward the latter, due to his being connected with a pole having a polarity opposing that of the screen 5, by separating from the node points 7 of the net element 6 and by impinging upon the patient at the region in which treatment has to be concentrated.

During treatment, the patient "P" may comfortably sit in front of the screen 5, while reading, listening to music or talking with other people, watching television of during normal work activities.

For this purpose, the wire 8 will have such a length as to allow any kind of movement, even when it is connected to the generator arrangement 2.

The skilled person may easily understand from the above description that the net element 6 may also have non-flat shapes, e.g. concave shapes mating with the convex profile of a part of the body of the patient "P".

The invention has been found to fulfill the intended objects.

The invention so conceived is susceptible to changes and variants within the inventive concept.

Also, all the details may be replaced by other technical equivalent elements.

In practice, any material, shape and size may be used as needed, without departure from the scope as defined by the following claims.

## Claims

1. A portable therapeutic device, comprising:
- a ions generator unit (2) configured to generate negative ions or anions;;
- an ions emitting apparatus (4) configured to emit said negative ions or anions toward the body of a patient (P) during a therapeutic treatment , said ion emitting apparatus (4) being connected to said ion generator unit (2) via a connecting wire (3) and forming a first pole having a negative polarity;
- a second pole having a positive polarity;
- wherein said ion emitting apparatus (4) comprises a net element (6) having meshes of net and nodes (7) for connecting said meshes, said nodes (7) being configured to form emitting points of flows of ions toward said second pole through the body of the patient (P);
- wherein said ion emitting apparatus (4) is configured to be placed at a distance from the body of the patient (P) ;
- wherein said ion emitting apparatus (4) further comprises two sheets (11) made of a plastic material which is permeable for said negative ions or anions,
- wherein said net element (6) is arranged between said two sheets (11)
**characterized in that**
the second pole Is configured to be connected to the body of the patient (P).

2. A device according to claim 1, wherein said net element (6) is substantially flat and defines a negative ions or anions emitting screen (5).

3. A device according to claim1, wherein said plastic material is choosen between ABS (Acrylonitrile-Butadiene-styrene) and Polystyrene, and wherein that plastic material is transparent, opaque or coloured.

4. A device according to anyone of preceding claims, wherein said net element (6) can be shaped according with pre-selected shapes chosen between flat shape or concave shape mating with convex profile of a part of the body of the patient (P).

5. A device according to anyone of preceding claims, wherein said emitting apparatus (4) has a screen (5) comprising said net element (6), wherein said screen (5) is provided with feet (10).

6. A device according to claim 1, wherein said second pole is configured to be connected to the body of the patient (P) by a cable or a wire (8).

7. A device according to claims 1 and 6, wherein it comprises a bracelet configured to be connected to the second pole by said cable or wire (8).

8. A device according to claim 1, wherein said net element is conductive.

9. A device according to claim 1, wherein meshes of the net element (6) are formed by intersecting wires.

## Patentansprüche

1. Eine tragbare therapeutische Vorrichtung mit
- einer Ionen-Erzeugereinheit (2), die ausgestaltet ist für die Erzeugung negativer Ionen oder Anionen,
- einem Ionen abgebenden Gerät (4), das ausgestaltet ist die negativen Ionen oder Anionen an den Körper eines Patienten (P) während einer therapeutischen Behandlung abzugeben, wobei das Ionen abgebende Gerät (4) verbunden ist mit der Ionen-Erzeugereinheit (2) über einen Verbindungsdraht (3) und einen ersten Pol mit einer negativen Polarität bildet,
- einem zweiten Pol mit einer positiven Polarität,
- wobei das Ionen abgebende Gerät (4) ein Netzelement (6) aufweist mit Netzmaschen und Knoten (7) zur Verbindung der Maschen und die Knoten (7) ausgebildet sind, Abgabepunkte zur Weiterleitung von Ionen zum zweiten Pol durch den Körper des Patienten (P) zu bilden,
- wobei das Ionen abgebende Gerät (4) ausgebildet ist, mit Abstand von dem Körper des Patienten (P) angeordnet zu werden,
den Körper des Patienten (P) zu bilden,
- wobei das Ionen abgebende Gerät (4) zudem zwei Lagen (11) aus Kunststoffmaterial aufweist, das durchlässig ist für die negativen Ionen oder Anionen,
- wobei das Netzelement (6) zwischen den zwei Lagen (11) angeordnet ist,
**dadurch gekennzeichnet, dass** der zweite Pol ausgebildet ist mit dem Körper des Patienten (P) verbunden zu werden.

2. Vorrichtung gemäß Anspruch 1, **dadurch gekennzeichnet, dass** das Netzelement (6) im Wesentlichen flach ist und eine negative Ionen oder Anionen abgebende Blende (5) bildet.

3. Vorrichtung gemäß Anspruch 1, **dadurch gekennzeichnet, dass** das Kunststoffmaterial ausgewählt ist zwischen ABS (Acrylonitrile-Butadiene-Styren) und Polystyren und wobei das Kunststoffmaterial durchsichtig, undurchsichtig oder farbig ist.

4. Vorrichtung gemäß einem der vorhergehenden Ansprüche, **dadurch gekennzeichnet, dass** das Netzelement (6) geformt sein kann entsprechend vorab ausgewählten Formen, die ausgewählt sind zwischen flacher Form oder konkaver Form passend zum konvexen Profil eines Teils des Körpers des Patienten (P).

5. Vorrichtung gemäß einem der vorhergehenden Ansprüche, **dadurch gekennzeichnet, dass** das Ionen abgebende Gerät (4) eine Blende (5) aufweist, die das Netzelement (6) umfasst, wobei die Blende (5) mit Füßen (10) versehen sind.

6. Vorrichtung gemäß Anspruch 1, **dadurch gekennzeichnet, dass** der zweite Pol ausgebildet ist über ein Kabel oder einen Draht (8) mit dem Körper des Patienten (P) verbunden zu werden.

7. Vorrichtung gemäß Anspruch 1 und 6, **dadurch gekennzeichnet, dass** ein Ring vorgesehen ist, der ausgebildet ist mit dem zweiten Pol über das Kabel oder den Draht (8) verbunden zu werden.

8. Vorrichtung gemäß Anspruch 1, **dadurch gekennzeichnet, dass** das Netzelement leitend ist.

9. Vorrichtung gemäß Anspruch 1, **dadurch gekennzeichnet, dass** Maschen des Netzelements (6) gebildet werden von sich überschneidenden Drähten.

## Revendications

1. Dispositif thérapeutique portable, comprenant :
une unité génératrice d'ions (2), conçue pour générer des ions ou des anions ;
- un appareil émetteur d'ions (4) conçu pour émettre lesdits ions négatifs ou anions vers le corps d'un patient (P) pendant un traitement thérapeutique, ledit dispositif émetteur d'ions (4) étant relié à ladite unité génératrice d'ions (2) au moyen d'un fil de connexion (3) et formant un premier pôle ayant une polarité négative ;
- un second pôle ayant une polarité positive ;
- dans lequel ledit appareil émetteur d'ions (4) comprend un élément en filet (6) ayant des mailles de filet et des noeuds (7) reliant lesdites mailles, lesdits noeuds (7) étant conçus pour former des points d'émission pour des flux ioniques vers ledit second pôle à travers ledit corps du patient (P) ;
- dans lequel ledit appareil émetteur de ions (4) est conçu pour être positionné à distance du corps du patient (P) ;
- dans lequel ledit appareil émetteur d'ions (4) comprend par ailleurs deux feuilles (11) en matière plastique perméable auxdits ions négatifs ou anions,
- dans lequel ledit élément en filet (6) est agencé entre lesdites deux feuilles (11)
caractérisé en ce
le second pôle est conçu pour être relié au corps du patient (P).

2. Dispositif selon la revendication 1, dans lequel ledit élément en filet (6) est sensiblement plat et définit un écran émetteur d'ions négatifs ou anions (5).

3. Dispositif selon la revendication 1, dans lequel ladite matière plastique est choisie parmi ABS (acrylonitrile-butadiéne-styrène) et polystyrène, et dans lequel la matière plastique est transparente, opaque ou colorée.

4. Dispositif selon n'importe laquelle des revendications précédentes, dans lequel ledit élément en filet (6) peut être façonné selon des profils présélectionnés, choisis entre une forme plate et une forme concave complémentaire à un profil convexe ou à une partie du corps du patient (P).

5. Dispositif selon n'importe laquelle des revendications précédents, dans lequel ledit dispositif émetteur (4) comporte un écran (5) comprenant ledit élément en filet (6), dans lequel ledit écran (5) est muni de pieds (10).

6. Dispositif selon la revendication 1, dans lequel ledit second pôle est conçu pour être relié au corps du patient (P) par un câble ou un fil (8).

7. Dispositif selon les revendications 1 et 6, dans lequel il comprend un bracelet conçu pour être relié au second pôle par ledit câble ou un fil (8).

8. Dispositif selon la revendication 1, dans lequel ledit élément en filet est conducteur.

9. Dispositif selon la revendication 1, dans lequel les mailles de l'élément en filet (6) sont formées par des fils croisés.
